# EUROPÄISCHE PATENTSCHRIFT

(11) **EP 1 387 824 B1**
(45) Veröffentlichungstag und Bekanntmachung des Hinweises auf die Patenterteilung: **06.06.2007**
(21) Anmeldenummer: 02735324.2
(22) Anmeldetag: 02.05.2002
(51) Int. Cl.: C07C 51/43, C07C 57/04, C07D 207/26

(54) **VERFAHREN DER KRISTALLISATIVEN REINIGUNG EINER ROH-SCHMELZE WENIGSTENS EINES MONOMEREN**
METHOD FOR PURIFYING A RAW MELT OF AT LEAST ONE MONOMER BY CRYSTALLIZATION
PROCEDE DE PURIFICATION PAR CRISTALLISATION D'UNE MASSE FONDUE BRUTE D'AU MOINS UN MONOMERE

(30) Priorität: 10.05.2001 DE 10122788
(43) Veröffentlichungstag der Anmeldung: 11.02.2004
(73) Patentinhaber: BASF Aktiengesellschaft, 67056 Ludwigshafen (DE)
(72) Erfinder: HAMMON, Ulrich, 68163 Mannheim (DE); ECK, Bernd, 68519 Viernheim (DE); BAUMANN, Dieter, 67227 Frankenthal (DE); HEILEK, Jörg, 69245 Bammental (DE); MÜLLER-ENGEL, Klaus, Joachim, 76297 Stutensee (DE)
(86) Internationale Anmeldenummer: PCT/EP2002/004783
(87) Internationale Veröffentlichungsnummer: WO 2002/090310

(56) Entgegenhaltungen:
- DE-A- 10 003 497

## Beschreibung

Vorliegende Erfindung betrifft ein Verfahren der kristallisativen Reinigung einer Roh-Schmelze wenigstens eines Monomeren, bei dem man die zu reinigende Roh-Schmelze einer Suspensionskristallisation unterwirft, die im Rahmen der Suspensionskristallisation erzeugten und in Restschmelze suspendierten Monomerenkristalle durch Anwendung einer ersten mechanischen Trennoperation von der Restschmelze abtrennt und in wenigstens einer weiteren Kristallisationsstufe die nach der ersten mechanischen Trennoperation verbliebene Restschmelze und/oder die abgetrennten, gegebenenfalls gewaschenen, Monomerenkristalle nach ihrem Aufschmelzen kristallisativ weiterreinigt.

Der Begriff Monomere soll in dieser Schrift chemische Verbindungen umfassen, die wenigstens eine ethylenisch ungesättigte Doppelbindung aufweisen.

Aufgrund der wenigstens einen ethylenisch ungesättigten Doppelbindung bilden Monomere äußerst reaktionsfähige Verbindungen, die unter anderem zur Herstellung von Polymerisaten Verwendung finden. Typische Beispiele für Monomere sind das N-Vinylpyrrolidon, die Acrylsäure, die Methacrylsäure und die Alkylester der vorgenannten Säuren.

Üblicherweise werden Monomere durch chemische Synthese aus geeigneten Rohstoffen erzeugt. Dabei werden sie, insbesondere aufgrund von bereits in den Rohstoffen enthaltenen Verunreinigungen und infolge unerwünschter Nebenreaktionen, normalerweise nicht unmittelbar in reiner Form befindlich sondern als Bestandteil von Mischungen erhalten, aus denen sie abgetrennt werden müssen. Historisch werden diesbezüglich als Trennverfahren insbesondere rektifikative oder extraktive und rektifikative Trennoperationen angewendet (vgl. z.B. EP-A 722926).

Nachteilig an diesen Trennverfahren ist, daß sie einen hohen Energieaufwand erfordern, da entweder unter hohen Rücklaufverhältnissen und/oder mit einer Vielzahl an theoretischen Trennstufen aufweisenden Rektifikationskolonnen gearbeitet werden muß.

Als Alternative gewinnt in jüngster Vergangenheit zunehmend die Verfahrensweise der Schmelzekristallisation zur Reinherstellung von Monomeren an Interesse (vgl. z.B. EP-A 616998).

Dabei wird als Ergebnis der Synthese sowie gegebenenfalls erster thermischer und/oder extraktiver Trennstufen eine Roh-Schmelze des wenigstens einen Monomeren erhalten. Darunter werden in dieser Schrift das wenigstens eine Monomere enthaltende Flüssigkeiten verstanden, die beim Abkühlen derselben als ersten Feststoff Kristalle des wenigstens einen Monomeren abscheiden, die weniger von dem wenigstens einen Monomeren verschiedene Substanzen enthalten als die Roh-Schmelze selbst.

D.h., der hier verwendete Begriff Roh-Schmelze trifft dann nicht, wenn sich beim Abkühlen anstelle des wenigstens einen Monomeren als erster Feststoff z.B. Extraktionsmittel oder eine andere Komponente abscheidet.

In der Regel enthalten die erfindungsgemäß wesentlichen Roh-Schmelzen des wenigstens einen Monomeren geringe Mengen-sogenannter Polymerisationsinhibitoren in Lösung befindlich (vgl. z.B. DE-A 19938841) zugesetzt, die eine unerwünschte radikalische Polymerisation des wenigstens einen Monomeren unter Einwirkung von Wärme und/oder Licht unterdrücken sollen.

Aus den wie vorstehend definierten Roh-Schmelzen des wenigstens einen Monomeren kann nun in an sich bekannter Weise durch Einwirkung von Kälte das wenigstens eine Monomere kristallisativ abgeschieden und so eine gereinigte Schmelze (in fester oder in flüssiger Form) des wenigstens einen Monomeren hergestellt werden (vgl. z.B. DE-A 19926082, WO 00/45928, WO 94/18166, DE-A 10026407, DE-A 10039025, DE-A 10003498 und DE-A 10003497).

Ganz generell wird dabei die verunreinigte Roh-Schmelze durch Abkühlen partiell erstarrt. Entsprechend dem Phasengleichgewicht weisen die entstehenden Monomerenkristalle einen geringeren verunreinigungsgehalt auf als die zurückbleibende flüssige Restschmelze. Abgeschwächt wird der vorstehend beschriebene, rein thermodynamisch bestimmte Trenneffekt durch Einschluß von Flüssigkeit während des Kristallisationsvorgangs und durch die dem Feststoff nach einer fest/flüssig-Trennung noch anhaftende Restschmelze.

Für das Erreichen hoher Reinheiten und/oder Ausbeuten sind daher oftmals mehrere aufeinanderfolgende Kristallisationsschritte (hier auch Kristallisationsstufen genannt) erforderlich. D.h., die in einer ersten Kristallisationsstufe gewonnenen Kristalle werden, gegebenenfalls nachdem sie mit einem geeigneten Lösungsmittel oder mit Schmelze von bereits gereinigten Kristallen zum Zweck der Entfernung von Restschmelze gewaschen wurden, wieder aufgeschmolzen und einem neuen Kristallisationsschritt unterworfen u.s.w..

Um die Ausbeute wirtschaftlich zu gestalten, wird auch die im ersten Kristallisationsschritt anfallende Restschmelze in der Regel wenigstens einem weiteren Kristallisationsschritt (in einer weiteren Kristallisationsstufe) unterworfen.

Generell können zur kristallisativen Reinigung von Roh-Schmelzen wenigstens eines Monomeren unterschiedliche Schmelzekristallisationsverfahren zur Anwendung kommen. Bei den Schichtkristallisationsverfahren wird das wenigstens eine Monomere in Form zusammenhängender, fest anhaftender Schichten ausgefroren.

Die fest/flüssig-Trennung erfolgt durch einfaches Abfließen lassen der Restschmelze. Anschließend kann das gereinigte Kristallisat aufgeschmolzen oder in einem gewollten Lösungsmittel zur weiteren Verwendung gelöst werden.

Pinzipiell unterscheidet man dabei zwischen "statischen" und "dynamischen" Schichtkristallisationsverfahren.

Bei den statischen Verfahren wird die zu reinigende Roh-Schmelze z.B. in Rohrbündel- oder modifizierte Plattenwärmeaustauscher eingefüllt und anschließend durch langsame Temperatursenkung auf der Sekundärseite teilweise erstarrt. Nach dem Ausfrieren wird die Restschmelze abgelassen und danach die abgeschiedene Kristallschicht als gereinigte Schmelze (Rein-Schmelze) abgeschmolzen (gegebenenfalls in Stufen). Der Wärme- und Stofftransport zu den Abscheide- bzw. Kristallschichtflächen erfolgt nur durch freie Konvektion.

Kennzeichnend für die dynamische Schichtkristallisation von Roh-Schmelzen ist eine erzwungene Konvektion der Roh-Schmelze. Diese kann durch Umpumpen der Roh-Schmelze durch volldurchströmte Rohre (z.B. DE-OS 2606364), durch Aufgabe der Roh-Schmelze als Rieselfilm (z.B. EP-A 616998) oder durch Einleiten von Inertgas in ein mit Schmelze gefülltes Rohr oder durch Pulsieren erfolgen.

Bei den Suspensionskristallisationsverfahren wird aus der Roh-Schmelze durch Kälteeinwirkung eine Kristallsuspension erzeugt, die die abgeschiedenen Kristalle in der Restschmelze suspendiert enthält. Dabei können die Feststoffkristalle unmittelbar in Suspension befindlich wachsen oder sich als Schicht auf einer gekühlten Wand abscheiden, von der sie anschließend abgekratzt und in der Restschmelze resuspendiert werden. Die Abtrennung der abgeschiedenen Kristalle von der Restschmelze wird bei einer Kristallsuspension üblicherweise durch eine mechanische Trennoperation (z.B. Abpressen, Filtrieren, Zentrifugieren und/oder in Waschkolonnen) vorgenommen.

Häufig wird nun für eine effiziente Abtrennung von wenigstens einem Monomeren aus Roh-Schmelzen empfohlen, verschiedene Kristallisationsmethoden kombiniert anzuwenden (vgl. z.B. EP-A 616998).

D.h., beispielsweise kann die Roh-Schmelze zunächst einer Suspensionskristallisation unterworfen werden. Die dabei abgeschiedenen, in der Restschmelze suspendierten Kristalle, werden dann durch eine mechanische Trennoperation von der Restschmelze abgetrennt und bilden entweder bereits das gewünschte reine Endprodukt oder werden, gegebenenfalls nachdem man sie mit z.B. Reinproduktschmelze gewaschen hat (z.B. durch Resuspension in selbiger), wieder aufgeschmolzen und in einer weiteren Kristallisationsstufe weitergereinigt. Diese weitere Kristallisationsstufe kann nun auch wieder eine Suspensionskristallisation, aber auch eine Schichtkristallisation sein. In entsprechender Weise kann die verbliebene Restschmelze in einer weiteren Kristallisationsstufe weitergereinigt werden. Diese kann zwar prinzipiell ebenfalls wieder eine Suspensionskristallisation aber auch eine Schichtkristallisation sein.

Charakteristisch für alle Kristallisationsverfahren ist, daß sie Engstellen, d.h. Stellen mit engem Strömungsquerschnitt aufweisen. So enthalten Fallfilmkristaller z.B. üblicherweise Einbauten, die nur einen kleinen Durchflußquerschnitt frei lassen. Das Überwinden dieser Engstelle gelingt der zu reinigenden Schmelze nur als dünner Film. Hinter der Engstelle bleibt dieser Film erhalten und fließt als Rieselfilm eine gekühlte Wandung herunter, auf der sich während des Fließprozesses Kristallisat abscheidet (vgl. z.B. EP-B 218545).

In anderer Weise erfolgt die Trennung einer Kristallsuspension in Kristalle und Restschmelze praktisch immer über Querschnitte, die nur für die Restschmelze nicht aber für die suspendierten Kristalle durchläßig sind (z.B. über ein zwei- oder dreidimensionales Netzwerk solcher Querschnitte beim Filtrieren oder in einer Siebzentrifuge).

Üblicherweise werden die kristallisativen Verfahren zur Reinigung einer Roh-Schmelze wenigstens eines Monomeren mehr oder weniger kontinuierlich (bzw. halbkontinuierlich) durchgeführt. Voraussetzung für hohe Raum-Zeit-Ausbeuten ist dabei, daß die beschriebenen engen Strömungsquerschnitte nicht blockiert werden.

Anders als beim in der WO 0045928 angewandten fraktionierten Schichtkristallisationsverfahren sollte beim in der Präambel skizzierten Verfahren, bei dem zunächst zwingend eine Suspensionskristallisationsstufe angewendet werden muß, in den nachfolgenden Kristallisationsstufen (die zur Weiterreinigung der Suspensionskristalle bzw. zur Weiterreinigung der Restschmelze angewendet werden) kein aus der vorhergehenden Suspensionskristallisationsstufe importiertes Feststoffproblem bestehen. Mit der mechanischen Abtrennung der Suspensionskristalle sollten auch andere beim Senken der Temperatur ausgefallene Feststoffe abgetrennt werden, so daß die verbleibende Restschmelze von fremden Feststoffen frei sein sollte.

Aber auch die wieder aufgeschmolzenen Suspensionskristalle sollten von Feststoffen frei sein, würden doch gegebenenfalls mitabgeschiedene Fremdfeststoffe beim Aufschmelzen gleichfalls wieder in Lösung gehen.

Bei der praktischen Durchführung von in der Präambel skizzierten verfahren zur kristallisativen Reinigung einer Roh-Schmelze wenigstens eines Monomeren traten in den auf die eingangs anzuwendende Suspensionskristallisationsstufe nachfolgenden Kristallisationsstufen zur Weiterreinigung von Restschmelze bzw. den Suspensionskristallen immer wieder in unerwarteter Weise solche Blockaden auf (insbesondere im Fall von Acrylsäure, Methacrylsäure und N-Vinylpyrrolidon). Dies insbesondere dann, wenn die charakteristische Länge der Engstellen ≤ 5 mm beträgt. Bemerkenswerterweise bestand das die Blockade verursachende Material nicht aus Kristallisat, da die Blockade durch Erwärmen über den Schmelzpunkt des Kristallisats hinaus im Regelfall nicht beseitigt werden konnte.

Die Aufgabe der vorliegenden Erfindung bestand daher darin, das in der Präambel beschriebene Verfahren der kristallisativen Reinigung einer Roh-Schmelze wenigstens eines Monomeren zu verbessern.

Demgemäß wurde ein Verfahren zur kristallisativen Reinigung einer Roh-Schmelze wenigstens eines Monomeren, bei dem man die zu reinigende Roh-Schmelze einer Suspensionskristallisation unterwirft, die im Rahmen der Suspensionskristallisation erzeugten und in Restschmelze suspendierten Monomerkristalle durch Anwendung einer ersten mechanischen Trennoperation von der Restschmelze abtrennt und in wenigstens einer weiteren Kristallisationsstufe die nach der ersten mechanischen Trennoperation verbliebene Restschmelze und/oder die abgetrennten, gegebenenfalls gewaschenen, Monomerenkristalle nach ihrem Aufschmelzen weiterreinigt, dadurch gekennzeichnet, daß die Restschmelze und/oder die wieder aufgeschmolzenen Monomerkristalle auf ihrem Weg in die wenigstens eine weitere Kristallisationsstufe wenigstens einer weiteren mechanischen fest/flüssig-Trennoperation unterworfen werden.

Hintergrund der beanspruchten Erfindung ist der überraschende Befund, daß z.B. die Restschmelzen, die im Rahmen des erfindungsgemäßen Verfahrens nach der Eingangs anzuwendenden Suspensionskristallisation abgetrennt werden nicht wirklich frei von Feststoffproblemen sind, sondern trotz des Einsatzes von Polymerisationsinhibitoren geringste Mengen an in disperser (teilweise kolloider) Verteilung befindlichem, in unerwünschter Weise gebildetem, visuell praktisch nicht wahrnehmbarem Polymerisat des wenigstens einen zu reinigenden Monomeren enthalten, das bei über längere Zeit mehr oder weniger kontinuierlich bzw. halbkontinuierlich durchgeführtem Reinigungsverfahren die Blockade bewirkt.

Besonders kritisch ist der geschilderte Sachverhalt dadurch, daß die Polymerisate häufig nicht besonders hochmolekular und deshalb vielfach klebrig sind.

Das erfindungsgemäße Verfahren eignet sich insbesondere dann, wenn der Schmelzpunkt des relevanten Monomeren ≤ 200°C, bevorzugt s 150°C und besonders bevorzugt ≤ 100°C bzw. 5 bis 20°C beträgt.

Es ist insbesondere zur Herstellung gereinigter Schmelzen von Acrylsäure, Methacrylsäure, Alkylestern der vorgenannten Säuren und N-Vinylpyrrolidon geeignet.

Als erfindungsgemäß anzuwendende erste mechanische Trennoperation kommen prinzipiell alle Trennoperationen in Betracht, die zur Abtrennung von Feststoffen von Flüssigkeiten geeignet sind. Dies sind z.B. filtrierende, sedimentierende und zentrifugierende Trennoperationen. Bevorzugt werden zentrifugierende Verfahren (z.B. Siebzentrifugen) und/oder Waschkolonnen (vgl. DE-A 10017903) angewendet.

Als erfindungsgemäß anzuwendende wenigstens eine weitere mechanische fest/flüssig-Trennoperation kommen prinzipiell ebenfalls alle Trennoperationen in Betracht, die zur Abtrennung von Feststoffen (insbesondere feinteiligen) von Flüssigkeiten geeignet sind. Besonders geeignet sind filtrierende und zentrifugierende Trennoperationen. Als Filtermaterialien können dabei Spaltsiebe, Lochsiebe, Siebgewebe, Filtervliese, Faserschichten, Fasergewebe, Sintermaterialien oder Schüttschichten (z.B. auch Sand) verwendet werden. Die Porengröße des vorstehend verwendeten Filtermaterials beträgt für die erfindungsgemäß anzuwendende wenigstens eine weitere mechanische fest/flüssig-Trennoperation in anwendungstechnisch zweckmäßiger Weise 50 bis 1000 µm. Häufig sollte es 100 bis 500 µm betragen. Vielfach ist aber auch ein Bereich von 10 bis 20 µm oder darunter anzuwenden. Die Filtration kann dabei als Druck- oder Vakuumfiltration praktiziert werden. Selbstverständlich kann sie auch als Siebzentrifugieren praktiziert werden. Sedimentierende Einrichtungen (Dekanter, Hydrozyklone, Lamellenklärer, Verweilzeitbehälter) sind für das erfindungsgemäße Verfahren für die wenigstens eine weitere mechanische Trennoperation weniger bevorzugt. Das Filter kann erfindungsgemäß auch unmittelbar im Einlaß zur wenigstens einen weiteren Kristallisationsstufe angebracht sein.

Das erfindungsgemäße Verfahren eignet sich insbesondere dann, wenn die Roh-Schmelze einer Suspensionskristallisation unterworfen und die resultierende Kristallsuspension wie in der DE-A 10039025 beschrieben mit Hilfe einer Waschkolonne in Restschmelze und Kristalle getrennt wird.

Dies gilt insbesondere dann, wenn das relevante Monomere Acrylsäure ist und auf dem Weg der heterogen katalysierten Gasphasenoxidation wie in der DE-A 19909923 beschrieben hergestellt worden ist. Erfindungsgemäß günstig ist es, wenn die nach Anwendung der ersten mechanischen Trennoperation verbliebene Restschmelze bevor sie der nächsten Kristallisationsstufe zugeführt wird wenigstens auf die Schmelztemperatur der reinen Kristalle erwärmt wird.

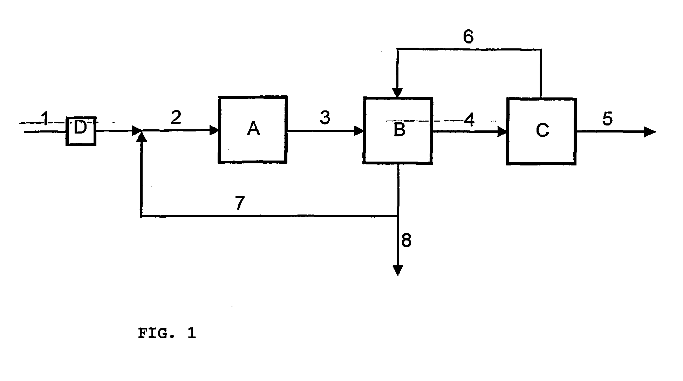

Beispiele und Vergleichsbeispiel (die im Beispiel verwendeten Adressen beziehen sich auf die beiliegende Figur 1)

Analog zu Beispiel 2 der DE-A 19909923 wurden durch fraktionierte Kondensation eines abgekühlten Produktgasgemisches einer zweistufigen heterogen katalysierten Gasphasenoxidation von Propen 340 kg/h einer Roh-Acrylsäure (Roh-Schmelze) des nachfolgenden Inhalts erzeugt:

| | |
|---|---|
| Acrylsäure | 97,3 Gew.-%, |
| Essigsäure | 0,8 Gew.-%, |
| Propionsäure | 500 Gew.-ppm, |
| Furfural | 700 Gew.-ppm, |
| Maleinsäureanhydrid | 40 Gew.-ppm, |
| Benzaldehyd | 200 Gew. -ppm, |
| Wasser | 1,3 Gew.-%, |
| Phenothiazin | 150 Gew.-ppm. |
| (Polymerisationsinhibitor) | |

Sie (1) wurde über ein Siebkorbfilter D gefahren und einem Suspensionskristallisator A zugeführt. Dazu wurden Chemienormpumpen vom Typ CPK (das sind Kreiselpumpen mit einer doppelten Gleitringdichtung) verwendet, wie sie beispielsweise die Firmen KSB oder Allweiler herstellten. Der Suspensionskristallisator war ein Kühlscheibenkristallisator (7 Kühlscheiben, insgesamt ca. 16 m² Kühlfläche, der Durchmesser der kreisrunden Kühlscheiben betrug 1,25 m, 2500 1 Innenvolumen). Die Zulauf temperatur der Roh-Acrylsäure betrug 25°C. Die Kristallisationswärme wurde über die Kühlflächen abgeführt. Die Restschmelze wurde beim Durchgang durch den Kühlscheibenkristallisator auf 7°C abgekühlt. Aus dem Suspensionskristallisator heraus wurde die Kristallsuspension, die einen Feststoffanteil von etwa 25 % ihres Gemisches aufwies, kontinuierlich auf eine 2-stufige Schubsiebzentrifuge B gegeben (Schubzentrifugen findet man z.B. beschrieben in der Broschüre WB 210/11.92AL der Fa. Siebtechnik, D-Mülheim an der Ruhr; bei einer 2-stufigen Schubsiebzentrifuge sind eine rotierende (größere) Außensiebtrommel (repräsentiert die zweite Stufe) und eine (kleinere) rotierende Innensiebtrommel (repräsentiert die erste Stufe) konzentrisch angeordnet; die Außensiebtromme1 führt nur Drehbewegungen aber keine Schubbewegung aus; die Innensiebtrommel läuft mit gleicher Drehzahl um wie die Außensiebtrommel und wird zusätzlich durch einen hydraulichen Schubkolben in axialer Richtung hin und her bewegt; beide Trommeln weisen zur Ableitung der Flüssigkeit Siebstruktur auf; in der Aufgabezone der Innensiebtrommel wird sofort der größte Teil der Restschmelze durch die Sieböffnungen abgeschleudert; der Feststoff bleibt als Filterkuchen auf dem Sieb zurück; beim axialen Rückgang der Innentrommel wird am freien Trommelende eine der Schublänge entsprechende Menge Feststoff in die Außensiebtrommel abgeworfen und dort weiter entfeuchtet; beim axialen Vorgehen der Innensiebtrommel wird der Filterkuchen schrittweise in die Außensiebtrommel (die in der Regel länger ist als die Innensiebtrommel) weitergeschoben und schließlich in eine Fangrinne abgeworfen), auf der das Suspensionskristallisat von der Restschmelze abgetrennt und zusätzlich mit einem Strom (6) gewaschen (Waschflüssigkeit war 23 kg/h 25°C aufweisendes, aufgeschmolzenes, gewaschenes Kristallisat) wurde. Der Innendurchmesser der ersten Stufe betrug 200 mm. Die Siebspaltweite der ersten Stufe betrug 250 µm. Die zweite Stufe war konisch gestaltet (der Innendurchmesser erweiterte sich von 250 mm auf 310 mm, die Siebspaltweite betrug 300 µm) . Die Drehzahl betrug 2200 Umdrehungen je Minute. Die Hubzahl der Innensiebtrommel lag bei 70 pro Minute.

Die abgeworfenen gewaschenen Kristalle (4) wurden in einem Behälter C aufgeschmolzen und 227 kg/h gereinigte Acrylsäure (5) mit einem Acrylsäuregehalt > 99 Gew. -% entnommen. Die in- Stufe-B abgetrennte Restschmelze wurde teilweise (113 kg/h) ausgeschleust (8), zum größten Teil (660 kg/h) aber unter Verwendung von Chemienormpumpen vom Typ CPK (das sind Kreiselpumpen mit doppelter Gleitringdichtung) wie sie beispielsweise die Firmen KSB oder Allweiler herstellen zum Suspensionskristallisator zurückgeführt (7) und mit dem Strom (1) hinter dem Siebkorbfilter D zum eine Temperatur von 15°C aufweisenden transparenten und optisch an Feststoff freien Strom (2) vereinigt, um einem weiteren Kristallisationsschritt zugeführt zu werden.

Nach etwa sechswöchigem Betrieb der Zentrifuge B kam es zum Überschiessen der Kristallsuspension auf der ersten Stufe, d.h., die Flüssigphase wurde nicht mehr ausreichend auf der ersten Stufe abgetrennt, und strömte kanalartig über den auf der ersten Stufe ausgebildeten Filterkuchen in die zweite Stufe. Dies ist insofern von Nachteil, als damit ein Anstieg der Restfeuchte des aus der zweiten Stufe abgeworfenen Kristallisats und ein unruhiger Lauf der Zentrifuge (bedingt durch die Unwucht aufgrund der Kanalbildung und Filterkuchendeformation auf der ersten Stufe) einhergeht, der zu einer Drehzahlreduktion zwingt.

Nach dem Einbau zweier Wechselfilter (Siebkorbfilter mit jeweils 550 cm² Siebfläche und 150 µm Siebweite) in den Rückführstrom 7 zum Suspensionskristallisator wurde unter ansonsten identischen Betriebsbedingungen auch nach mehr als viermonatiger Betriebsdauer kein Überschiessen der Suspension auf der ersten Stufe festgestellt.

Ein Wechsel und Reinigen der Wechselfilter erfolgte dabei in etwa wöchentlichem Abstand. Es befand sich darin gummiartiges, klebriges Polymer, das sich als Polyacrylsäure erwies und mit wäßriger Natronlauge und nachfolgend Wasser ausgewaschen werden konnte.

## Patentansprüche

1. Verfahren der kristallisativen Reinigung einer Roh-Schmelze wenigstens eines Monomeren, bei dem man die zu reinigende Roh-Schmelze einer Suspensionskristallisation unterwirft, die im Rahmen der Suspensionskristallisation erzeugten und in Restschmelze suspendierten Monomerenkristalle durch Anwendung einer ersten mechanischen Trennoperation von der Restschmelze abtrennt und in wenigstens einer weiteren Kristallisationsstufe die nach der ersten mechanischen Trennoperation verbliebene Restschmelze und/oder die abgetrennten, gegebenenfalls gewaschenen, Monomerenkristalle nach ihrem Aufschmelzen weiterreinigt, **dadurch gekennzeichnet, daß** die Restschmelze und/oder die wieder aufgeschmolzenen Monomerenkristalle auf ihrem Weg in die wenigstens eine weitere Kristallisationsstufe wenigstens einer weiteren mechanischen fest/flüssig-Trennoperation unterworfen werden.

2. Verfahren nach Anspruch 1, **dadurch gekennzeichnet, daß** die kristallisative Weiterreinigung der Restschmelze durch Suspensionskristallisation erfolgt.

3. Verfahren nach Anspruch 1, **dadurch gekennzeichnet, daß** die kristallisative Weiterreinigung der Restschmelze durch Fallfilmkristallisation erfolgt.

4. Verfahren nach einem der Ansprüche 1 bis 4, **dadurch gekennzeichnet, daß** das wenigstens eine Monomere ausgewählt ist aus der Gruppe umfassend Acrylsäure, Methacrylsäure und N-Vinylpyrrolidon.

## Claims

1. A process for the purification of a crude melt of at least one monomer by crystallization, in which the crude melt to be purified is subjected to a suspension crystallization, the monomer crystals produced during the suspension crystallization and suspended in residual melt are separated from the residual melt by using a first mechanical separation operation and, in at least one further crystallization stage, the residual melt remaining after the first mechanical separation operation and/or the monomer crystals which have been separated off and if appropriate washed are further purified after they have been melted, wherein the residual melt and/or the remelted monomer crystals are subjected to at least one further mechanical solid/liquid separation operation on their way into the one or more further crystallization stages.

2. The process according to claim 1, wherein the further purification of the residual melt by crystallization is effected by suspension crystallization.

3. The process according to claim 1, wherein the further purification of the residual melt by crystallization is effected by falling-film crystallization.

4. The process according to any of claims 1 to 4, wherein the one or more monomers are selected from the group consisting of acrylic acid, methacrylic acid and N-vinylpyrrolidone.

## Revendications

1. Procédé de purification par cristallisation d'une masse fondue brute d'au moins un monomère, dans lequel on soumet la masse fondue brute à purifier à une cristallisation en suspension, on sépare les cristaux monomères produits dans le cadre de la cristallisation en suspension et suspendus dans la masse fondue résiduelle par application d'une première opération de séparation mécanique de la masse fondue résiduelle et, dans au moins une étape de cristallisation supplémentaire, on continue à purifier la masse fondue résiduelle subsistant après la première opération de séparation mécanique et/ou les cristaux monomères séparés, éventuellement lavés après leur fusion, **caractérisé en ce que** la masse fondue résiduelle et/ou les cristaux monomères refondus sont soumis, au cours de l'au moins une étape de cristallisation supplémentaire à au moins une autre opération de séparation mécanique solide/liquide.

2. Procédé selon la revendication 1, **caractérisé en ce que** la purification par cristallisation supplémentaire de la masse fondue résiduelle a lieu par cristallisation en suspension.

3. Procédé selon la revendication 1, **caractérisé en ce que** la purification par cristallisation supplémentaire de la masse fondue résiduelle a lieu par cristallisation par film tombant.

4. Procédé selon l'une des revendications 1 à 4, **caractérisé en ce qu'**au moins un monomère est sélectionné à partir du groupe comprenant l'acide acrylique, l'acide méthacrylique et la N-vinyle-pyrrolidone.
